(19) Europäisches Patentamt
European Patent Office
Office européen des brevets

(11) Publication number: **0 299 680 A2**

(12) **EUROPEAN PATENT APPLICATION**

(21) Application number: **88306208.5**

(51) Int. Cl.4: **A61K 31/445**

(22) Date of filing: **07.07.88**

(30) Priority: **10.07.87 GB 8716338**

(43) Date of publication of application: **18.01.89 Bulletin 89/03**

(84) Designated Contracting States: **AT BE CH DE FR GB IT LI LU NL SE**

(71) Applicant: **NATIONAL RESEARCH DEVELOPMENT CORPORATION**
**101 Newington Causeway**
**London SE1 6BU(GB)**

(72) Inventor: **Costall, Brenda**
**The Old Rectory**
**Addingham North Yorkshire(GB)**

(74) Representative: **Burford, Anthony Frederick et al**
**W.H. Beck, Greener & Co. 7 Stone Buildings**
**Lincoln's Inn**
**London WC2A 3SZ(GB)**

(54) **Anti-anxiogenic compositions containing dioxopiperidine derivatives.**

(57) Phenyl-3-aminoalkyl-4-methyl-2,6-dioxopiperidines of the Formula I

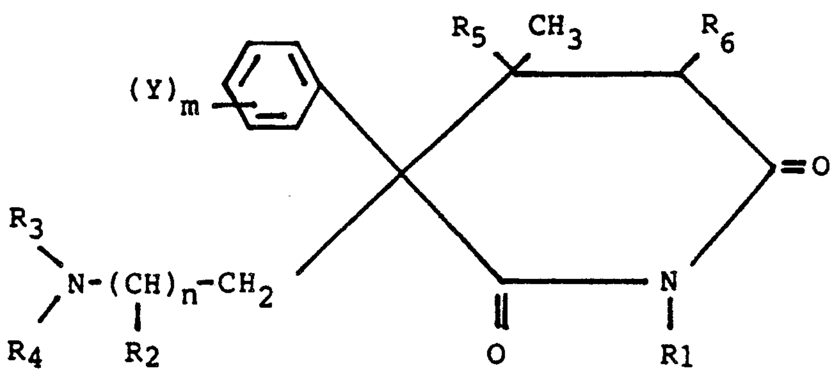

(I)

wherein:

$R_1$ represents hydrogen or $C_1$-$C_4$ alkyl;
n is 1 or 2;
$R_2$ represents hydrogen or methyl, provided that one $R_2$ is hydrogen when n is 2;
$R_3$ represents hydrogen or $C_1$-$C_2$ alkyl;
$R_4$ represents $C_1$-$C_2$ alkyl;
$R_5$ and $R_6$ independently represent hydrogen or methyl;
m is 0 to 3; and
each Y is in a meta or para position and independently represents hydroxy, $C_1$-$C_2$ alkoxy, $C_1$-$C_2$ alkyl, $C_1$-$C_2$ hydroxyalkyl, halogen, or trifluoromethyl, provided that hydroxy and alkoxy are not in the para position,

EP 0 299 680 A2

and pharmacologically acceptable salts thereof antagonise anxiogenesis associated with withdrawal from addictive drugs, especially alcohol, cocaine and nicotine.

The presently preferred compound is 3-(3′methoxyphenyl)-3-(3″-N,N-dimethylaminopropyl) -4,4-dimethyl-2,6-dioxopiperidine (AGN 2979).

# ANTI-ANXIOGENIC COMPOSITIONS CONTAINING DIOXOPIPERIDINE DERIVATIVES

This invention relates to the use of certain 3-phenyl-3-aminoalkyl-4-methyl-2,6-dioxopiperidines to antagonise anxiogenesis associated with withdrawal from drug addiction. In particular, the invention provides the use of the said dioxopiperidines in the manufacture of medicaments for the treatment of said anxiogenesis and methods of treatment of said anxiogenesis using said dioxopiperidines.

It has been recognised in recent years that anxiogenesis is a substantial problem associated with the withdrawal from drug addiction. Many addictive drugs, such as alcohol, nicotine and cocaine, are anxiolytic and when the drug is abruptly withdrawn anxiogenesis usually develops. The severity and duration of the anxiogenesis generally depends upon the duration of the addiction. Anxiogenesis also is encountered with withdrawal from narcotic and other drugs producing physical dependance.

Benzodiazepines agonists especially diazepam, have been used to treat anxiogenesis associated with drug, especially alcohol, withdrawal. However, sedative, rather than anti-anxiety, doses have been employed and treatment often results in substitution of the original drug addiction for benzodiazepine addiction. Anxiolytic drugs generally have not been considered of use in the treatment of anxiogenesis associated with addictive drug withdrawal because of the substantial dose levels required and the risk of substitute addiction.

It has surprisingly now been found that certain 3-phenyl-3-aminoalkyl-4-methyl-2,6-dioxopiperidines (as defined hereinafter) potently antagonise anxiogenesis associated with addictive drug withdrawal.

GB 1455687 (also AU 480855, BE 808958, DE 23630526, FR 7346904, JP 6053014 and US 3963729) discloses that 3-phenyl-3-aminoalkyl-4- and/or 5-methyl-2,6-dioxopiperidine derivatives have central nervous system, especially antidepressant, activity. Said compounds include, inter alia, those of the following Formula A.

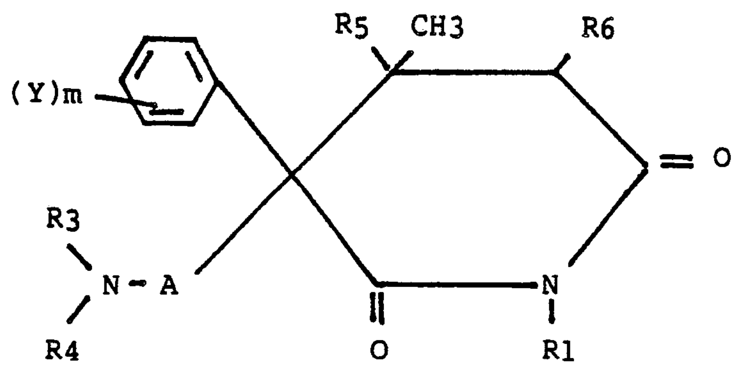

(A)

wherein:

R1 represents hydrogen or $C_1$-$C_4$ alkyl;

R3 represents hydrogen or $C_1$-$C_4$ alkyl;

R4 represents $C_1$-$C_4$ alkyl;

R5 and R6 independently represent hydrogen or methyl;

A represents $C_1$-$C_6$ alkylene;

m is 0 to 3; and

Y is hydroxy, $C_1$-$C_4$ alkoxy, $C_1$-$C_4$ alkyl, $C_1$-$C_4$ hydroxyalkyl, halogen or trifluoromethyl.

The dose level specified for administration of the compounds is 0.1 to 100 mg/kg using pharmaceutical compositions containing 1 to 1000 mg per unit dose.

It also has been disclosed in U.S. 4,461,771 that compounds of Formula A, in which $R_1$ represents hydrogen; $R_3$ and $R_4$ independently represent methyl or ethyl; $R_5$ represents methyl; $R_6$ represents hydrogen; A represents ethylene or propylene; m is 1 or 2; and each Y is in a meta position and independently represents hydroxy or $C_1$-$C_2$ alkoxy, are believed to reduce in vitro the activity of tryptophan hydroxylase by blocking the depolarization-induced activation of the enzyme in the brain stem and hence are of potential use in the prophylactic treatment of the stressful disorder migraine. The dose level specified for this treatment is 0.01 to 10 mg/kg, especially 0.1 to 3 mg/kg, using pharmaceutical compositions containing 0.1 to 200 mg, usually 1 to 100 mg, per unit dose. More recently, it has been reported that at least one compound of Formula A (viz 3-(3′-methoxy-phenyl)-3- (3″-N,N-dimethylaminopropyl)-4,4-dimethyl-2,6-dioxopiperidine; AGN 2979) also blocks in vitro the activation of tryptophan hydroxylase resulting from exposure of brain stem slices to metabolic inhibitors or methylxanthines or induced by incubation of supernatant preparations of the enzyme under phosphorylating conditions (Boadle-Biber, M.C. et al Biochem. Pharmacol. 35, 1521-6, (1986)). However, it also has been reported that AGN 2979 has no significant effect in vitro upon the unactivated enzyme (Boadle-Biber, M.C. et al supra).

Further, it has recently been disclosed in GB 2181346A that compounds of Formula A, in which $R_1$ represents hydrogen; $R_3$ and $R_4$ independently represent methyl or ethyl; A represents ethylene or propylene; m is 1 or 2; and each Y is in a meta position and independently represents hydroxy or $C_1$-$C_2$ alkoxy, are believed to reduce the turnover of 5-hydroxytryptamine (5HT) resulting from inhibiting the activity of tryptophan hydroxylase. They are reported to have anxiolytic activity, antagonize the anxiogenic activity of benzodiazepines inverse agonists, reduce chronic abnormally high brain levels of 5HT or its metabolite 5-hydroxy-indoleacetic acid, and have antibacterial and antiviral activity.

G.B. 2181346A was published in pursuance of U.K. Patent Application No. 8621577 filed 8th September 1986 and claiming priority from U.K. Patent Applications Nos. 8522455 (filed 11th September 1985), 8603909 (filed 17th February 1986) and 8603910 (also filed 17th February 1986). Originally, it was thought that the disclosed compounds were not themselves anxiolytic because they have virtually no action at benzodiazepine receptors and that they acted via some unknown pharmacological mechanism to potentiate the anxiolytic activity of benzodiazepine receptors. Their anxiolytic activity was disclosed for the first time in U.K. Patent Application No. 8621577. At that time, the compounds were believed to be active in the range 0.1 to 20 mg/kg, especially 0.5 to 10 mg/kg and hence pharmaceutical compositions containing 10 to 500 mg, especially 10 to 100 mg, were proposed. It was subsequently surprisingly found that the compounds are active at much lower dose levels, down to nanogram/kg amounts.

According to a first aspect of the present invention, there is provided the use in the manufacture of a medicament for the treatment of anxiogenesis associated with addictive drug withdrawal of a compound of the following Formula I.

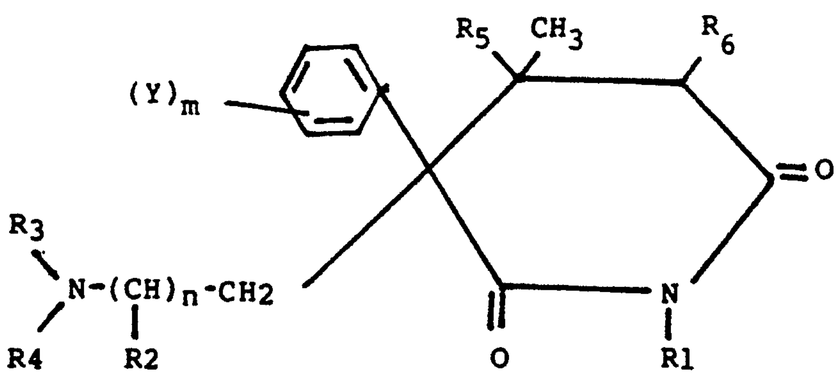 (I)

wherein:

$R_1$ represents hydrogen or $C_1$-$C_4$ alkyl; n is 1 or 2;

$R_2$ represents hydrogen or methyl, provided that one $R_2$ is hydrogen when n is 2;

n is 1 or 2;

$R_2$ represents hydrogen or methyl, provided that one $R_2$ is hydrogen when n is 2;

$R_3$ represents hydrogen or $C_1$-$C_2$ alkyl;

$R_4$ represents $C_1$-$C_2$ alkyl;

$R_5$ and $R_6$ independently represent hydrogen or methyl;

m is 0 to 3; and

each Y is in a meta or para position and independently represents hydroxy, $C_1$-$C_2$ alkoxy, $C_1$-$C_2$ alkyl, $C_1$-$C_2$ hydroxyalkyl, halogen, or trifluoromethyl, provided that hydroxy and alkoxy are not in the para position,

or a pharmacologically acceptable salt thereof.

In a second aspect, the invention provides a method of treating a patient suffering from anxiogenesis associated with addictive drug withdrawal, which comprises administering to the patient an anti-anxiogenic effective amount of a compound of the following Formula I.

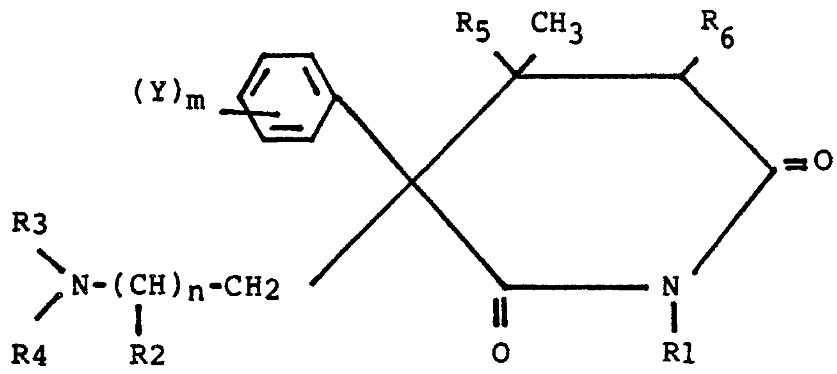

(I)

wherein:

$R_1$ represents hydrogen or $C_1$-$C_4$ alkyl;

n is 1 or 2;

$R_2$ represents hydrogen or methyl, provided that one $R_2$ is hydrogen when n is 2;

$R_3$ represents hydrogen or $C_1$-$C_2$ alkyl;

$R_4$ represents $C_1$-$C_2$ alkyl;

$R_5$ and $R_6$ independently represent hydrogen or methyl;

m is 0 to 3; and

each Y is in a meta or para position and independently represents hydroxy, $C_1$-$C_2$ alkoxy, $C_1$-$C_2$ alkyl, $C_1$-$C_2$ hydroxyalkyl, halogen, or trifluoromethyl, provided that hydroxy and alkoxy are not in the para position,

or a pharmacologically acceptable salt thereof.

The compounds of Formula I can be prepared in the manner disclosed in GB 1455687. They exist as optical isomers and can be used in racemate form or as individual (+) or (-) isomers. Presently, the (-) isomer is preferred.

As mentioned above the compounds of Formula 1 antagonise anxiogenesis associated with addictive drug withdrawal and hence are useful in the treatment of said withdrawal. Unlike benzodiazepine agonists, they do not cause sedation at high doses ($10^2$ mg/kg). In the treatment of withdrawal from alcohol, nicotine, cocaine and other addictive drugs not causing physical dependence, they can be used alone. However, in the treatment of narcotic and the like physically addictive drugs, they will be used with drugs required to treat the physical aspects of the drug withdrawal.

The compounds of Formula I can be administered in various manners to achieve the desired anxiogenic effect. The compounds can be administered enterally or parenterally to the patient being treated. Oral administration is likely to be the preferred route in most circumstances but injection, especially subcutaneously or intravenously, will be preferred in some circumstances.

The amount of compound administered will vary and can be any anti-anxiogenic effective amount. Depending upon the patient and the mode of administration, the amount of compound administered may vary over a wide range to provide from about $10^{-7}$ to $10^2$ mg/kg, usually $10^{-5}$ to $10^2$ mg/kg, especially $10^{-4}$ to $10^2$ mg/kg, of body weight of the patient per unit dose. Unit doses of these compounds can contain, for example, from about $10^{-6}$ mg to 500 mg, usually $10^{-4}$ to $10^2$ mg, especially $10^{-3}$ to $10^2$ mg of the compound and may be administered, for example, from 1 to 4 times daily.

The compounds of Formula I have virtually no action at benzodiazepine receptors. The capacity of a selected number of compounds of Formula I to displace triturated flunitrazepam from benzodiazepine receptors has been measured with the results set forth in Table I below:-

TABLE 1

| | COMPOUND OF FORMULA I | | | | |
|---|---|---|---|---|---|
| | 2979 | 3222 | 2939 | 3181 | DIAZEPAM* |
| $IC_{50}$(uM) [$^3$H]Flunitrazepam Binding | 350 | 1300 | 9000 | 6700 | 0.014 |

* 7-chloro-1,3-dihydro-1-methyl-5-phenyl-2H-1,4-benzodiazepine-2-one

The term "unit dosage form" is used herein to mean a single or multiple dose form containing a quantity of the active ingredient in admixture with or otherwise in association with a diluent or carrier, said quantity being such that one or more predetermined units are normally required for a single therapeutic administration. In the case of multiple dose forms such as liquids or scored tablets, said predetermined unit will be one fraction, such as a 5 ml (teaspoon) quantity of a liquid or a half or quarter of a scored tablet, of the multiple dose form.

The compounds of general Formula I can have the phenyl moiety substituted in one or both meta positions by $C_1$-$C_2$ alkyl, $C_1$-$C_2$ hydroxyalkyl, halogen, trifluoromethyl, or, preferably, hydroxy or $C_1$-$C_2$ alkoxy. Additionally or alternatively, the phenyl moiety can be substituted in the para position by the aforementioned groups other than hydroxy and alkoxy. It is presently preferred that the substituent(s) should be hydroxy or, especially, methoxy. It is also preferred that one or both meta positions are substituted and that, when there are two substituents, they should be the same.

The amino group of the compounds of Formula I can be secondary or tertiary having methyl or ethyl groups attached to the nitrogen atom. Dimethylamino presently is preferred. The amino group is connected to the piperidine ring by a divalent ethylene (i.e. n = 1) or trimethylene (i.e. n = 2) radical optionally substituted on a carbon atom not adjacent said ring with a methyl group. Presently, unsubstituted trimethylene is preferred.

The piperidine ring of the compounds of Formula I is substituted in the 4-position with methyl and optionally by one or two further methyl groups in the 4 and/or 5 positions. It is presently preferred that there is one further methyl group in the 4 or 5 position, especially in the 4-position.

The ring nitrogen atom of the piperidine ring can be substituted with a $C_1$-$C_4$ alkyl group but it is presently preferred that said nitrogen atom is unsubstituted.

The $C_1$-$C_2$ alkyl groups or moieties referred to herein are methyl or ethyl; methyl presently being preferred. The $C_3$-$C_4$ alkyl groups which may be substituents on the nitrogen atom of the piperidine ring can be straight or branched chain but the straight chain n-propyl or n-butyl groups presently are preferred. The halogen substituent(s) in the phenyl ring can be chlorine, bromine or fluorine; chlorine presently being preferred.

The presently preferred compounds of Formula I are those of the following Formula IA.

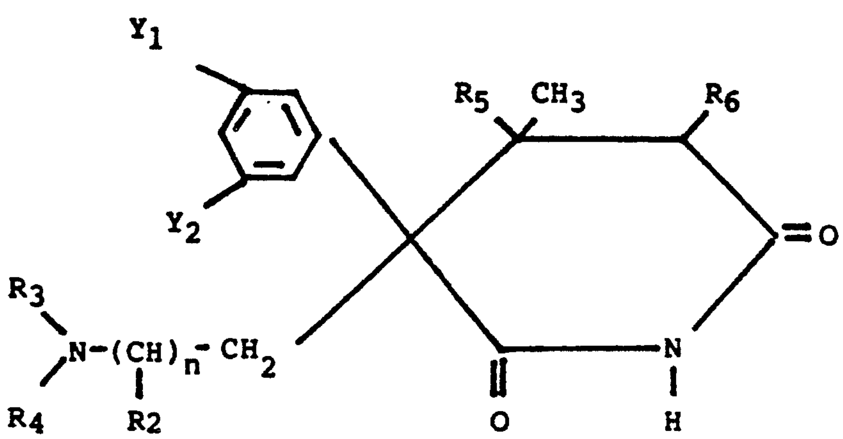

(IA)

wherein:

n is 1 or 2;

$R_2$ represents hydrogen or methyl, provided that one $R_2$ is hydrogen when n is 2;

$R_3$ represents hydrogen or $C_1$-$C_2$ alkyl;

$R_4$ represents $C_1$-$C_2$ alkyl;

$R_5$ and $R_6$ independently represent hydrogen or methyl; and

$Y_1$ and $Y_2$ independently represent hydrogen, hydroxy or $C_1$-$C_2$ alkoxy,

or a pharmacologically acceptable salt thereof.

The presently especially preferred compounds of Formula 1A are those of the following Formula IB.

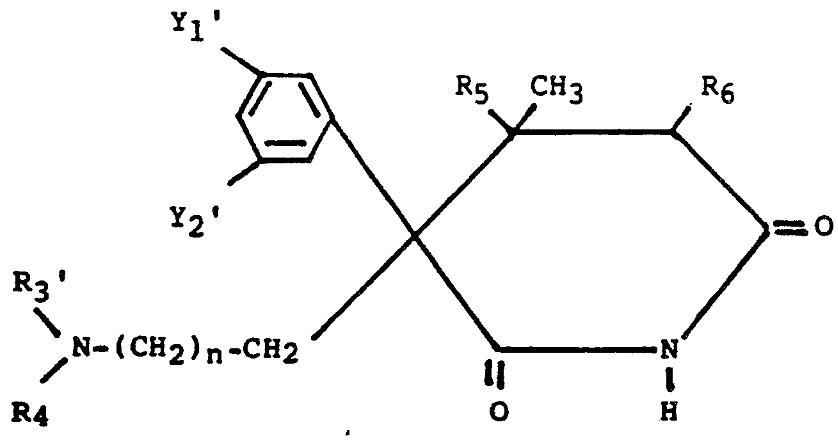

(IB)

wherein:

$\underline{n}$ is 1 or 2;

$R_3'$ and $R_4$ independently represent $C_1$-$C_2$ alkyl;

$R_5$ and $R_6$ independently represent hydrogen or methyl;

$Y_1'$ represents hydroxy or $C_1$-$C_2$ alkoxy; and

$Y_2'$ represents hydrogen, hydroxy or $C_1$-$C_2$ alkoxy,

or a pharmacologically acceptable salt thereof.

Examples of compounds of Formula IC include the following:-

3-(3′-methoxyphenyl)-3-(2″-N,N-dimethylaminoethyl)-4,4-dimethyl-2,6-dioxopiperidine
3-(3′-methoxyphenyl)-3-(3″-N,N-dimethylaminopropyl)-4,4-dimethyl-2,6-dioxopiperidine (AGN 2979);
3-(3′-methoxyphenyl)-3-(2″-N,N diethylaminoethyl)-4,4-dimethyl-2,6-dioxopiperidine;
3-(3′-methoxyphenyl)-3-(3″-N,N-diethylaminopropyl)-4,4-dimethyl-2,6-dioxopiperidine;
3-(3′-hydroxyphenyl)-3-(2″-N,N-dimethylaminoethyl)-4,4-dimethyl-2,6-dioxopiperidine;
3-(3′-hydroxyphenyl)-3-(3″-N,N-dimethylaminopropyl)-4,4-dimethyl-2,6-dioxopiperidine;
3-(3′-methoxyphenyl)-3-(2″-N,N-dimethylaminoethyl)-4,5-dimethyl-2,6-dioxopiperidine (AGN 2939);
3-(3′-methoxyphenyl)-3-(3″-N,N-dimethylaminopropyl)-4,5-dimethyl-2,6-dioxopiperidine (AGN 3181);
3-(3′-ethoxyphenyl)-3-(3″-N,N-dimethylaminopropyl)-4,4-dimethyl-2,6-dioxopiperidine;
3-(3′-ethoxyphenyl)-3-(3″-N,N-diethylaminopropyl)-4,4-dimethyl-2,6-dioxopiperidine;
3-(3′,5′-dimethoxyphenyl)-3-(3″-N,N-dimethylaminopropyl)-4,4-dimethyl-2,6-dioxopiperidine (AGN 3222);
3-(3′,5′-dimethoxyphenyl)-3-(2″-N,N-dimethylaminoethyl)-4,4-dimethyl-2,6-dioxopiperidine;
3-(3′,5′-dimethoxy phenyl)-3-(3″-N,N-dimethylaminopropyl)-4,5-dimethyl-2,6-dioxopiperidine; and
3-(3′,5′-dimethoxyphenyl)-3-(2″-N,N-dimethylamioethyl)-4,5-dimethyl-2,6-dioxopiperidine;

Examples of other compounds of Formula I include:-

3-phenyl-3-(2′-N,N-dimethylaminoethyl)-4-methyl-2,6-dioxopiperidine;
3-phenyl-3-(2′-N,N-dimethylaminoethyl)-4,4-dimethyl-2,6-dioxopiperidine;
3-phenyl-3-(2′-N,N-dimethylaminoethyl)-4,5-dimethyl-2,6-dioxopiperidine;
3-phenyl-3(3′-N,N-dimethylaminopropyl)-4,4-dimethyl-2,6-dioxopiperidine;
3-(4′-chlorophenyl)-3(3″-N,N-dimethylaminopropyl)-4,4-dimethyl-2,6-dioxopiperidine; and
3-phenyl-3(2′ N-methylaminoethyl)-4,4-dimethyl-2,6-dioxopiperidine.

The compounds of Formula I may be administered in free base form, as an alkali metal or alkaline earth metal salt or as a pharmaceutically acceptable acid addition salt with the proviso that an alkali metal or alkaline earth metal salt is not normally combined with an acid addition salt except in a layer formulation. Representative acid addition salt forms include organic acid salt forms such as the maleate and methane sulphonate and mineral acid salt forms such as the hydrochloride and perchlorate.

The pharmaceutical formulations in which form the active compounds of the invention will normally be utilized are prepared in a manner well known *per se* in the pharmaceutical art and usually comprise at least one active compound of Formula I in admixture or otherwise in association with a pharmaceutically acceptable carrier or diluent therefor. For making those formulations the active ingredient will usually be mixed with a carrier, or diluted by a diluent, or enclosed or encapsulated in a capsule, sachet, cachet, paper or other container. A carrier or diluent may be solid, semi-solid or liquid material which serves as a vehicle, excipient or medium for the active ingredient. Suitable carriers or diluents are well known *per se*.

The formulations may be adapted for enteral or parenteral use and may be administered to the patient in the form of tablets, capsules, dragees, suppositories, syrups, suspensions, subcutaneous or intramuscular

depot injections or implants or the like. The formulations may be in delayed or sustained release form.

Aside from the active agents the compositions may contain pharmaceutically inert organic or inorganic adjuvants, optionally granulating agents, binding agents lubricants, dispersing agents, wetting agents and preservatives. Moreover, the pharmaceutical compositions may contain colouring, flavouring and sweetening substances. Adjuvants for the production of tablets may be e.g. calcium carbonate, lactose micro-crystalline cellulose, mannitol or talc. Starch and alginic acid or micro-crystalline cellulose may be used as granulating and disintegrating agents, starch, polyvinylpyrrolidone and gelatine may be used as binding agents and magnesium stearate, stearic acid, colloidal silica and talc as lubricants. Tablet formulation may be coated or uncoated, the coating having the purpose of delaying the disintegration and absorption in the gastrointestinal tract. Suitable suspending agents for the production of liquid administration forms are e.g. methyl cellulose and sodium alginate. Capsule formulation may contain the active agents on their own or together with an inert solid diluent e.g. calcium phosphate, corn starch, lactose, or mannitol.

The invention is illustrated in the following non-limiting Examples.

## EXAMPLE 1

### Tablet Formulation

Tablets each having the following composition are prepared by conventional techniques:

| | mg/tablet |
|---|---|
| (a) Compound AGN 2979 base | 1 |
| (b) Lactose | 51.5 |
| (c) Maize starch dried | 45 |
| (d) Magnesium stearate | 1.5 |

## EXAMPLE 2

### Suppository Formulation

| | mg/suppository |
|---|---|
| (a) Compound AGN 2979 HCl | 10 |
| (b) Oil of Theobroma (cocoa butter) | 990 |

The compound (a) is powdered and passed through a BS No. 100 sieve and triturated with molten oil of Theobroma at 45 °C to form a smooth suspension. The mixture is well stirred and poured into moulds each of nominal 1 G capacity to produce suppositories.

## EXAMPLE 3

Tablet Formulation

(a) Compound AGN 2979 base 10mg
(b) Wheat starch 7g
(c) Lactose 20g
(d) Magnesium Stearate 1g

EXAMPLE 4

Pill Formulation

| | per pill |
|---|---|
| (a) Compound AGN 2979 HCl | 10mg |
| (b) Corn starch | 45mg |
| (c) Liquid glucose | 7ml |

The pills are prepared by blending the active ingredient (a) and the corn starch, then adding the liquid glucose with thorough kneading to form a plastic mass from which the pills are cut and formed.

EXAMPLE 5

Gelatine Capsule Formulation

| | per capsule |
|---|---|
| (a) Compound AGN 2979 HCl | 2.5mg |
| (b) Talc | 70mg |

A capsule is prepared by passing dry powdered active ingredient (a) and powdered talc in the above proportions through a fine mesh screen and mixing them well. The powder is then filled into hard gelatin capsules at a net fill of 72.5mg per capsule.

EXAMPLE 6

Naive male albino BKW mice, 25-30g, were used in all experiments. 10 mice were normally housed in each cage and kept on a 12 h light/dark cycle with lights off at 08.00h.

The apparatus used for the detection of changes in exploratory behaviour consisted of an open-topped box (45 x 27 x 27 cm high) lined into a 9 cm squares, one third painted black and illuminated under a dim red light (1 x 60W) and partitioned from the remainder of the box which was painted white and brightly illuminated with a 100W light source located 17 cm above the box. An opening 7.5 x 7.5 cm located at floor level in the centre of the partition allowed access between the black and white areas.

Tests were conducted between 13.00 and 18.00h in a quiet darkened room illuminated with a red light. Animals which had been subject to alcohol (8% w/v in drinking water) were taken for testing either during alcohol intake or following its withdrawal with or without concomitant drug (diazepam or AGN 2979) treatment. Mice were taken from a dark holding room in a dark container to the dark testing room where, after a period of adaptation to the environment of the test room, they were placed individually into the centre of the white, brightly-lit area of the test box and their behaviour observed over a 5 min period by remote video recording. The behaviours noted were (a) the number of exploratory rearings in the white and black sections, (b) the number of line crossings in the white and black areas, (c) the number of transitions between the white and black or black and white areas, (d) the time spent in the white and black areas and (e) latency of movement from the white to black area.

The anxiogenesis of alcohol withdrawal is maximum at 24 and 48h following removal of alcohol intake. Diazepam (10 mg/kg i.p.) or AGN 2979 (0.5 mg/kg i.p.) were given at the time of alcohol withdrawal and then b.d. until testing 48h after alcohol withdrawal.

During the intake of alcohol, mice exhibit marked anxiolysis characterised by increased exploratory behaviour in the white section of the test box, with corresponding decreases in the black (Figure 1). The anxiolysis is also seen in the delayed latency for movement from the white to the black, and a reduction in the % of time spent in the black. When the alcohol is withdrawn, a reverse behavioural profile of anxiogenesis is observed. This becomes apparent within 24h of withdrawal and persists for longer than 48h. The intensity of this anxiogenesis of withdrawal from alcohol is exemplified by the data given on Figure 1 showing markedly reduced activity in the white section of the test box, with correspondingly marked elevations in exploratory activity in the black.

The anxiogenesis of alcohol withdrawal can be antagonised by diazepam (10 mg/kg given b.d., Figure 2) or by AGN 2979 (0.5 mg/kg given b.d., Figure 3). It is interesting that relatively large doses of anxiolytic agents are required to antagonise the alcohol withdrawal anxiogenesis, and that treatment should ideally commence at the time of withdrawal with subsequent b.d. dosing (during this time tolerance rapidly develops to the sedative actions of diazepam).

It can be seen from Figures 2 and 3 that, when effective, both diazepam and AGN 2979 not only reverse the anxiogenesis but actually lead to anxiolysis. Diazepam and AGN 2979 appear to be equieffective to combat the anxiogenesis of alcohol withdrawal, but AGN 2979 is considerably more potent and is devoid of the initial sedative actions seen on treatment with diazepam.

## EXAMPLE 7

The procedure of Example 6 was repeated with mice given nicotine (0.1 mg/kg b.d.) for 14 days. They were tested during nicotine intake and then 8, 48 and 96 h after nicotine withdrawal. The number of exploratory rearings and the number of line crossings in the black and white areas were noted. Anxiogenesis of withdrawal was marked at 48 h and the actions of diazepam and AGN 2979 were determined at this time. The diazepam and AGN 2979 were given at the time of nicotine withdrawal and then b.d. until testing. The mice were used once only and each testing time utilised naive animals in groups of 5-10.

Figure 4 shows the anxiolytic action of nicotine (0.1 mg/kg i.p. b.d. 14 days) and anxiogenesis following withdrawal of the nicotine treatment in mice tested in the black:white box. C indicates the response of vehicle-treated mice (responses indistinguishable from those of normal, untreated mice). n = 6. S.E.M.s less than 11.13%, *,$^{+}$P less than 0.001 (Student's t test).

Figure 5 shows the antagonism of nicotine (Nic.) withdrawal (W/D) anxiogenesis (0.1 mg/kg b.d. 7 days, 48 h W/D) by diazepam (1.0 mg/kg i.p.) in the mouse. C = control, Nic. = during nicotine treatment, Nic. W/D = during nicotine withdrawal, Nic. W/D + Diaz = diazepam given during nicotine withdrawal. n = 6. S.E.M.s less than 12.7%, *,$^{+}$P less than 0.01 - P less than 0.001 (compared to C), $^{\bullet}$P less than 0.001 (reversal of withdrawal)(Student's t test).

Figure 6 shows the antagonism of nicotine withdrawal (W/D) anxiogenesis (0.1 mg/kg b.d. 7 days, 48 h W/D) by AGN 2979 (1.0 mg/kg s.c.) in the mouse. C = control, Nic. = during nicotine treatment, Nic. W/D = during nicotine withdrawal Nic. W/D + BTG = AGN 2979 given during nicotine withdrawal. n = 6. S.E.M.s less than 11.2%, *,$^{+}$P less than 0.001 (compared to C), $^{\bullet}$P less than 0.001 (reversal withdrawal)-(Student's t test).

Anxiolysis presented throughout a 14 day period of treatment of mice with nicotine, 0.1 mg/kg b.d. This was revealed in the black:white test box as a reduced aversion to the white, brightly-lit environment (increased exploratory rearings and line crossings in the white, correspondingly decreased in the black, Fig.

4). This anxiolysis was reversed to anxiogenesis 8 - 96 h after withdrawing the nicotine treatment. This was seen in the black:white test box as increased exploratory rearings and line crossings in the black, dimly-lit area, with corresponding reductions in behaviour in the white compartment (Fig. 4).

The anxiogenesis of nicotine withdrawal could be antagonised by treatments with diazepam or AGN 2979 (Figs. 5 and 6). Both compounds were effective at 1 mg/kg at which doses the anxiogenesis was reversed to anxiolysis. However, at this dose level diazepam causes sedation but AGN 2979 does not.

## EXAMPLE 8

The procedure of Example 7 was repeated with mice given cocaine (1.0 mg/kg b.d.) for 14 days. As in said Example, the mice were treated at cocaine intake and at 8, 48 and 96 h after cocaine withdrawal. On this occasion, anxiogenesis of cocaine withdrawal was marked at 8 h and the actions of diazepam and AGN 2979, given at the time of cocaine withdrawal, were determined at this time.

Figure 7 shows the anxiolytic action of cocaine (1.0 mg/kg b.d. 14 days) and anxiogenesis following its abrupt withdrawal, measured using the mouse black:white test box. n = 6. S.E.M.s less than 11.9%. *, $^{+}$P less than 0.05 - P less than 0.001 (Student's t test, comparsion to C, control).

Figure 8 shows the antagonism of cocaine withdrawal (W/D) anxiogenesis in the mouse (1.0 mg/kg b.d. 14 days, W/D 8 h) by diazepam (10 mg/kg) as measured in the black:white test box. C = control, Coc. = during cocaine, Coc. W/D = during cocaine withdrawal, Coc. W/D + Diaz. = during cocaine withdrawal but with diazepam administered at the time of withdrawal. n = 6. S.E.M.s less than 12.7%. *, $^{+}$P less than 0.001 (compared to C). $^{\circ}$P less than 0.001 (reversal W/D)(Student's t test).

Figure 9 shows the antagonism of cocaine withdrawal (W/D) anxiogenesis in the mouse (1.0 mg/kg b.d. 14 days, W/D 8 h) by AGN 2979 (1 mg/kg s.c.) as measured in the mouse black:white test box. C = control, Coc. = during cocaine, Coc. W/D = during cocaine withdrawal, Coc. W/D + BTG = during cocaine withdrawal but with AGN 2979 administered at the time of withdrawal. n = 6. S.E.M.s less than 12.1%, *, $^{+}$P less than 0.001 (compared to C). $^{\circ}$P less than 0.001 (reversal W/D) (Student's t test).

In the mouse, long-term treatment with cocaine caused anxiolysis, after an initial stage of anxiogenesis on day 1. The anxiolysis was apparent on days 3 - 14 of treatment (Fig. 7). Anxiogenesis followed the withdrawal of treatment with cocaine, and was detected 8 - 96 h following withdrawal (Fig. 7).

The anxiogenesis of cocaine withdrawal was antagonised by diazepam at 10 mg/kg (Fig. 8) and by AGN 2979 at 1 mg/kg (Fig. 9). However, at these dose levels diazepam causes sedation but AGN 2979 does not.

## EXAMPLE 9

Male laboratory bred common marmosets (Callithrix jacchus), weighing between 350-400g, were housed in single sex pairs and allowed food (mazuri primate diet S.D.S. Ltd., Essex) ad libitum. Once daily marmosets were also given an assortment of fruit and brown or malt bread. Holding rooms were maintained at 25 ± 1 °C at a humidity of 55% and on a 12 h light/dark (red illumination) cycle (with simulated dawn and twilight periods) with lights on at 07.00 h.

Tests were conducted between 13.00 - 15.30 h in the normal holding room (to avoid unwanted disruption of behaviour by movement to a novel room or cage). The holding cages, in which the marmosets were housed individually, measured 76 cm high, 50 cm wide and 60 cm deep. "Anxiety" was initiated by a human observer standing 0.6 m in front of the holding cage. Changed behaviour was recorded over a 10 minute period (consecutive 2 minute recordings for each behavioural response(s) were measured) both by the observer and by blind assessment by an independent observer of video recordings taken throughout the period of human threat. The behavioural measures noted included (a) time spent on cage front in direct confrontation with the human threat, (b) the number of aggressive body postures, primarily shown as raising of the tail to expose the genital region with varying degrees of body piloerection, anal scent marking and slit stare with flattened ear tufts, (c) time spent in nest box avoiding confrontation with the human observer (threat) and (d) number of jumps to and from the cage front which indicates investigation of the situation and reduced apprehension.

The marmosets were given nicotine (0.01 mg/kg s.c. b.d.) for 7 days. They were tested on day 5 of nicotine intake and then 48 h after nicotine withdrawal. Anxiogenesis of withdrawal was marked at 48 h and the actions of AGN 2979 were determined at this time (a single dose given 40 min before testing).

Figure 10 shows the antagonism of nicotine (Nic.) withdrawal (W/D) anxiogenesis (0.01 mg/kg s.c. b.d. 7 days, 48h) by AGN 2979 (0.01 mg/kg s.c., 40 min treatment) in the marmoset. Nic = during nicotine treatment (day 5), Nic. W/D = during nicotine withdrawal, Nic. W/D + BTG = AGN 2979 given during nicotine withdrawal. n = 3. S.E.M.s 11 - 23%. *,⁺P less than 0.05 - P less than 0.001 (compared to C), •P less than 0.001 (reversal withdrawal) (Student's t test).

During nicotine treatment marmosets, similarily to mice, exhibited reduced anxiety responding. In the marmoset human threat test this was seen as reduced time spent in the nest box avoiding the human threat, as reduced numbers of aggressive postures and increased numbers of jumps to the front of the cage to confront the human threat (Fig. 10). Again, as in the mouse, anxiogenesis followed the withdrawal of treatment of marmosets with nicotine. In the marmoset this was seen as reduced time spent on the cage front confronting the observer, increased time in the nest box avoiding the observer and increased numbers of aggressive postures (Fig. 10). These effects of withdrawal from nicotine were all significantly antagonised by AGN 2979 at a dose of 0.01 mg/kg s.c. Indeed, this treatment not only antagonised the anxiogenesis of nicotine withdrawal but reversed this to anxiolysis seen as reduced time spent in the nest box, reduced numbers of aggressive postures, and increased numbers of jumps to and from the cage front to confront the human threat (Fig. 10).

## Claims

1. The use in the manufacture of a medicament for the treatment of anxiogenesis associated with addictive drug withdrawal of a compound of the following Formula I.

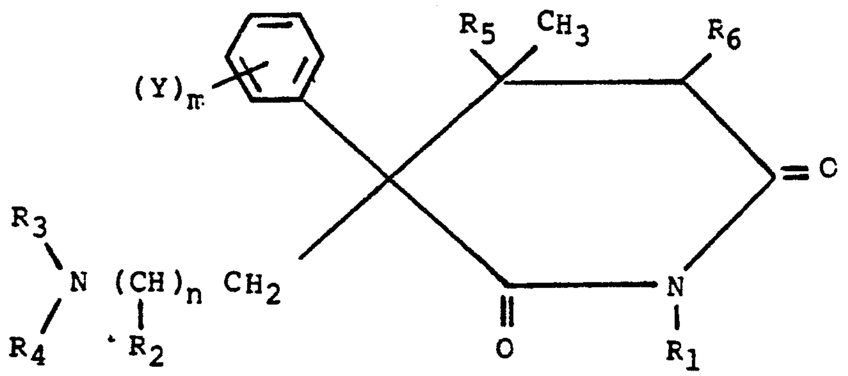

(I)

wherein:

$R_1$ represents hydrogen or $C_1$-$C_4$ alkyl;

$\underline{n}$ is 1 or 2;

$R_2$ represents hydrogen or methyl, provided that one $R_2$ is hydrogen when $\underline{n}$ is 2;

$R_3$ represents hydrogen or $C_1$-$C_2$ alkyl;

$R_4$ represents $C_1$-$C_2$ alkyl;

$R_5$ and $R_6$ independently represent hydrogen or methyl;

$\underline{m}$ is 0 to 3; and

each Y is in a meta or para position and independently represents hydroxy, $C_1$-$C_2$ alkoxy, $C_1$-$C_2$ alkyl, $C_1$-$C_2$ hydroxyalkyl, halogen, or trifluoromethyl, provided that hydroxy and alkoxy are not in the para position,

or a pharmacologically acceptable salt thereof.

2. A use as claimed in Claim 1, wherein the compound has the following Formula IA.

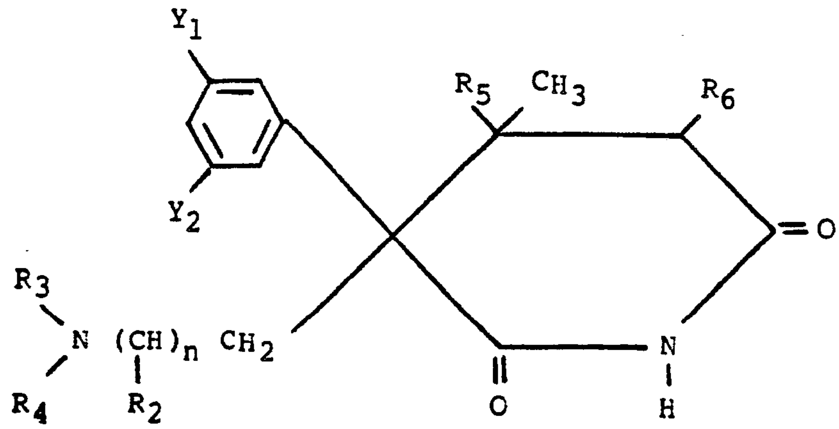

(IA)

wherein:

$\underline{n}$ is 1 or 2;

$R_2$ represents hydrogen or methyl, provided that one $R_2$ is hydrogen when $\underline{n}$ is 2;

$R_3$ represents hydrogen or $C_1$-$C_2$ alkyl;

$R_4$ represents $C_1$-$C_2$ alkyl;

$R_5$ and $R_6$ independently represent hydrogen or methyl; and

$Y_1$ and $Y_2$ independently represent hydrogen, hydroxy or $C_1$-$C_2$ alkoxy,

or a pharmacologically acceptable salt thereof.

3. A use as claimed in Claim 2, wherein the compound has the following Formula IB.

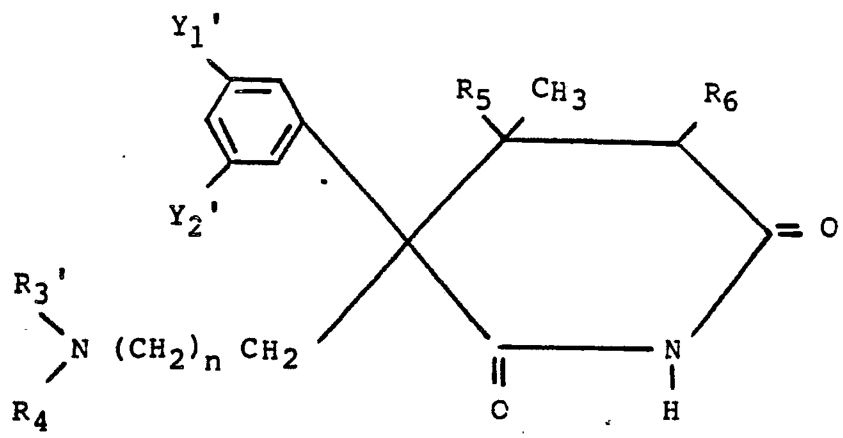

(IB)

wherein:

$\underline{n}$ is 1 or 2;

$R_3'$ and $R_4$ independently represent $C_1$-$C_2$ alkyl;

$R_5$ and $R_6$ independently represent hydrogen or methyl;

$Y_1'$ represents hydroxy or $C_1$-$C_2$ alkoxy; and

$Y_2'$ represents hydrogen, hydroxy or $C_1$-$C_2$ alkoxy,

or a pharmacologically acceptable salt thereof.

4. A use as claimed in Claim 3, wherein the compound is 3-(3′-methoxyphenyl)-3-(3″-N,N-dimethylaminopropyl) -4,4-dimethyl-2,6-dixopiperidine.

5. A use as claimed in Claim 4, wherein the compound is the minus isomer.

6. A use as claimed in any one of the preceding claims, wherein the medicament is in unit dosage form and contains $10^{-6}$ to 500 mg of the said compound of Formula I.

7.. A use as claimed in any one of the preceding claims, wherein the medicament is in unit dosage form and contains $10^{-4}$ to $10^{2}$ mg of the said compound of Formula 1.

8. A use as claimed in any one of the preceding claims, wherein the medicament is in unit dosage form and contains $10^{-3}$ to $10^{-1}$ mg of the said compound of Formula 1.

9. A use as claimed in any one of the preceding claims, wherein the said addictive drug is alcohol.

10. A use as claimed in any one of Claims 1 to 8, wherein the said addictive drug is nicotine.

11. A use as claimed in any one of Claims 1 to 8, wherein the said addictive drug is cocaine.

Neu eingereicht / Newly filed
Nouvellement déposé
(R 35)

FIG. 1.

Antagonism of withdrawal anxiogenesis [8% w/v, for 14 days, 48h W/D] by diazepam [10 mg/kg i.p.]

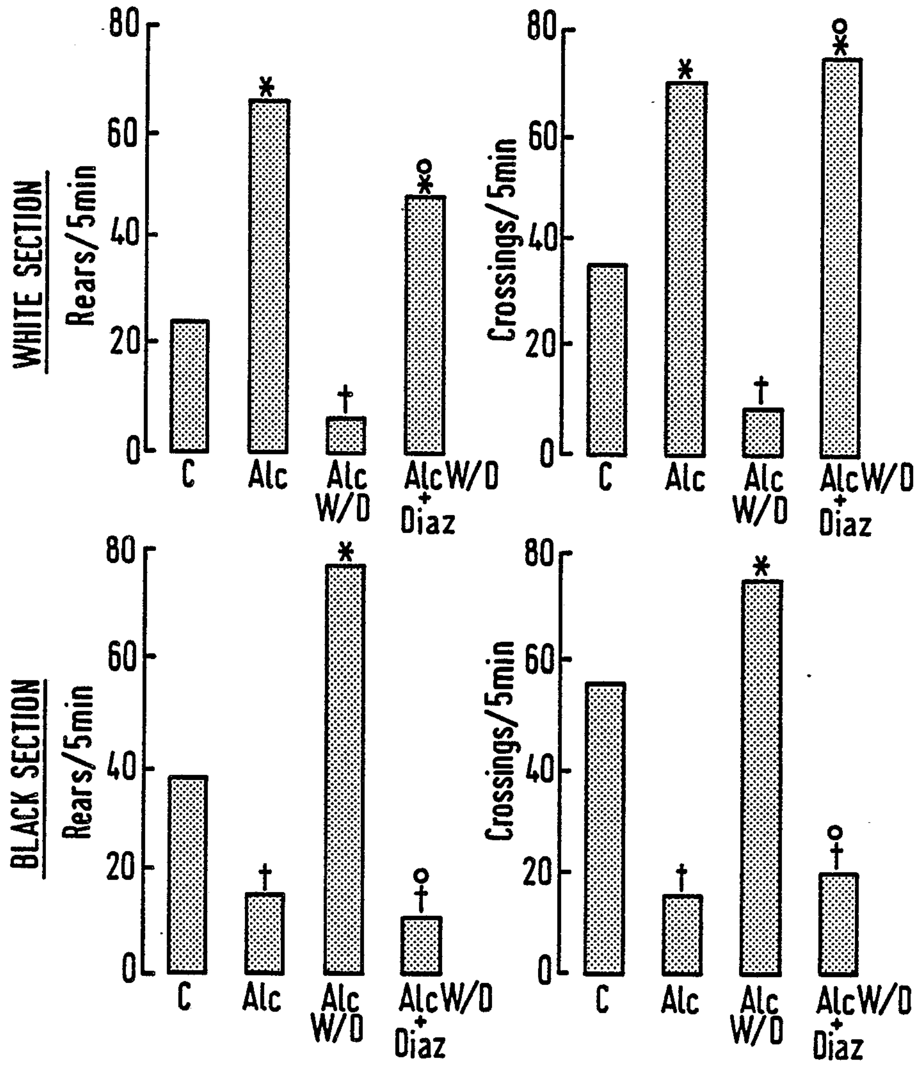

FIG. 2.

Anxiolytic action of alcohol and anxiogenesis following its abrupt withdrawal

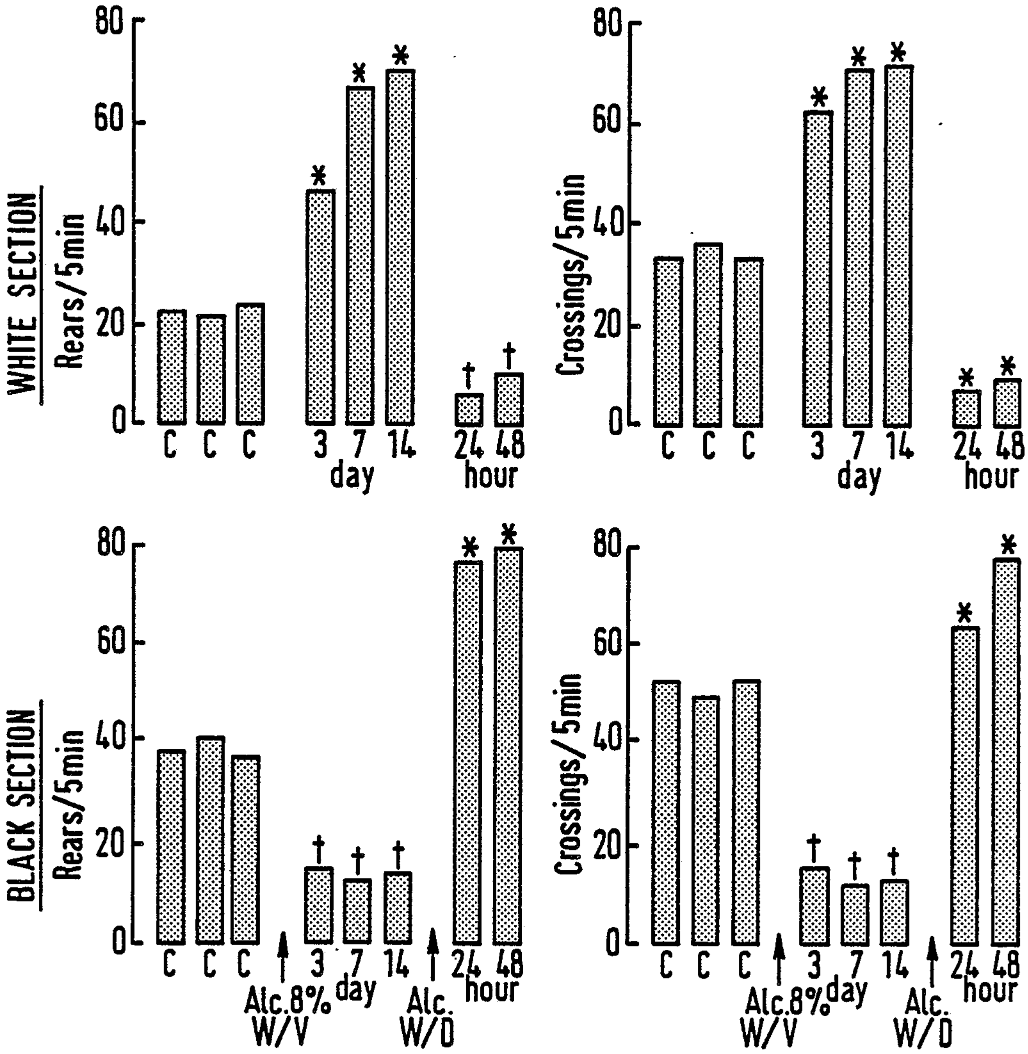

FIG. 3.

Antagonisom of alcohol withdrawal anxiogenesis [8%o w/v, 14days, 48h W/D] by BTG 1501 [0·5mg/kg i.p.].

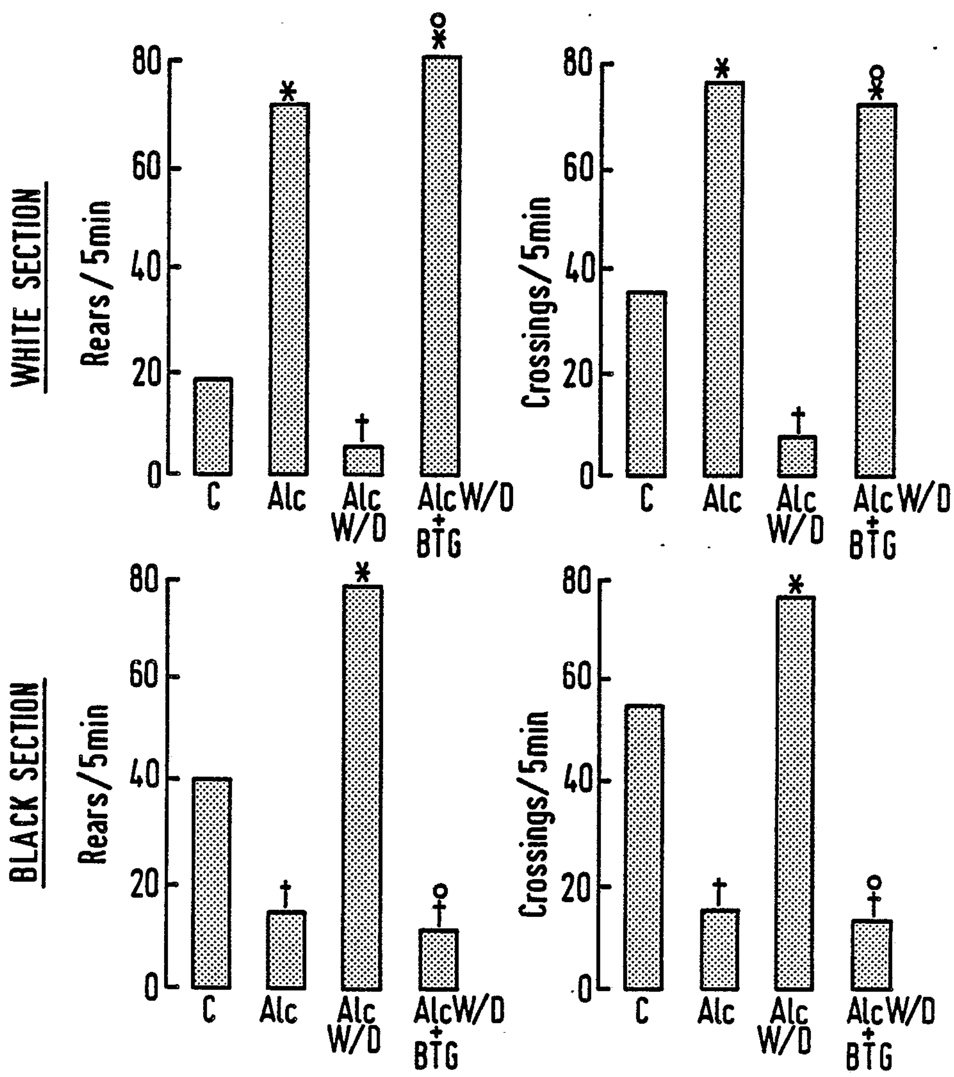

Neu eingereicht / Newly filed
Nouvellement déposé
(R 35)



FIG. 4.

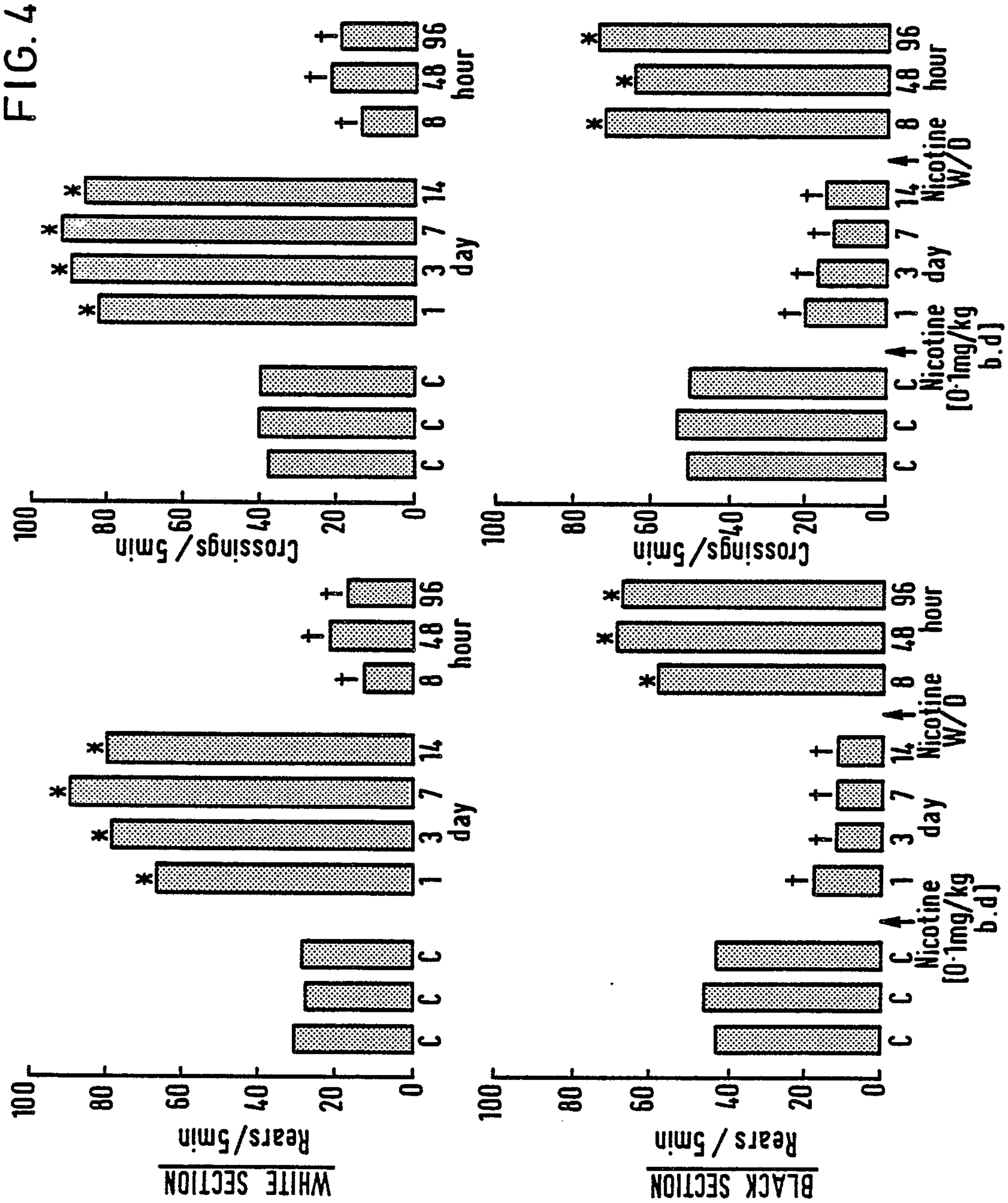

Nouvellement déposé
(R 35)

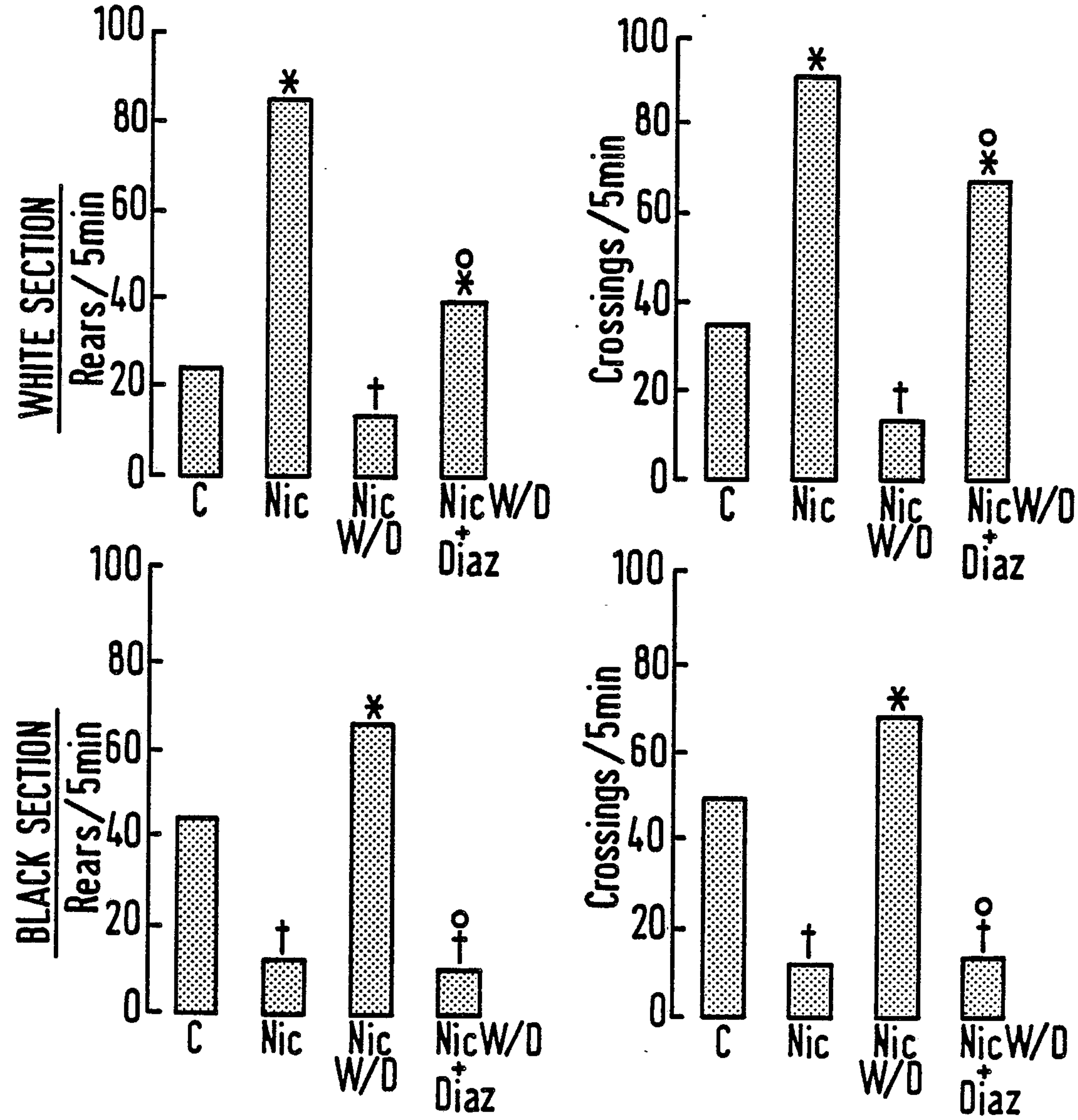


FIG. 5.

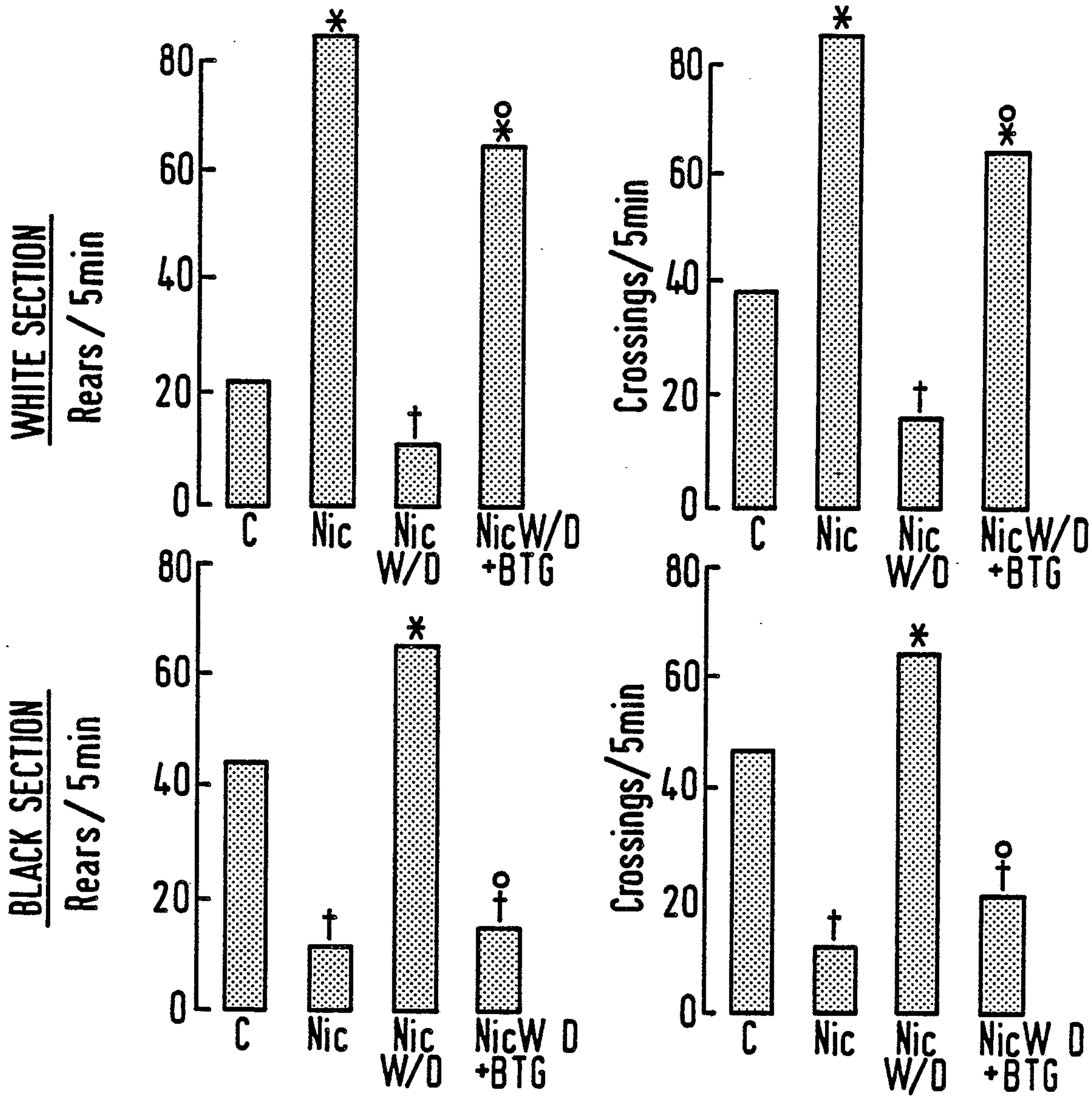
WHITE SECTION
Rears / 5min
0
20
40
60
80
C
Nic
Nic W/D
NicW/D +BTG
Crossings/5min
BLACK SECTION
Rears/ 5min
NicW D +BTG

FIG. 6.

FIG. 7.

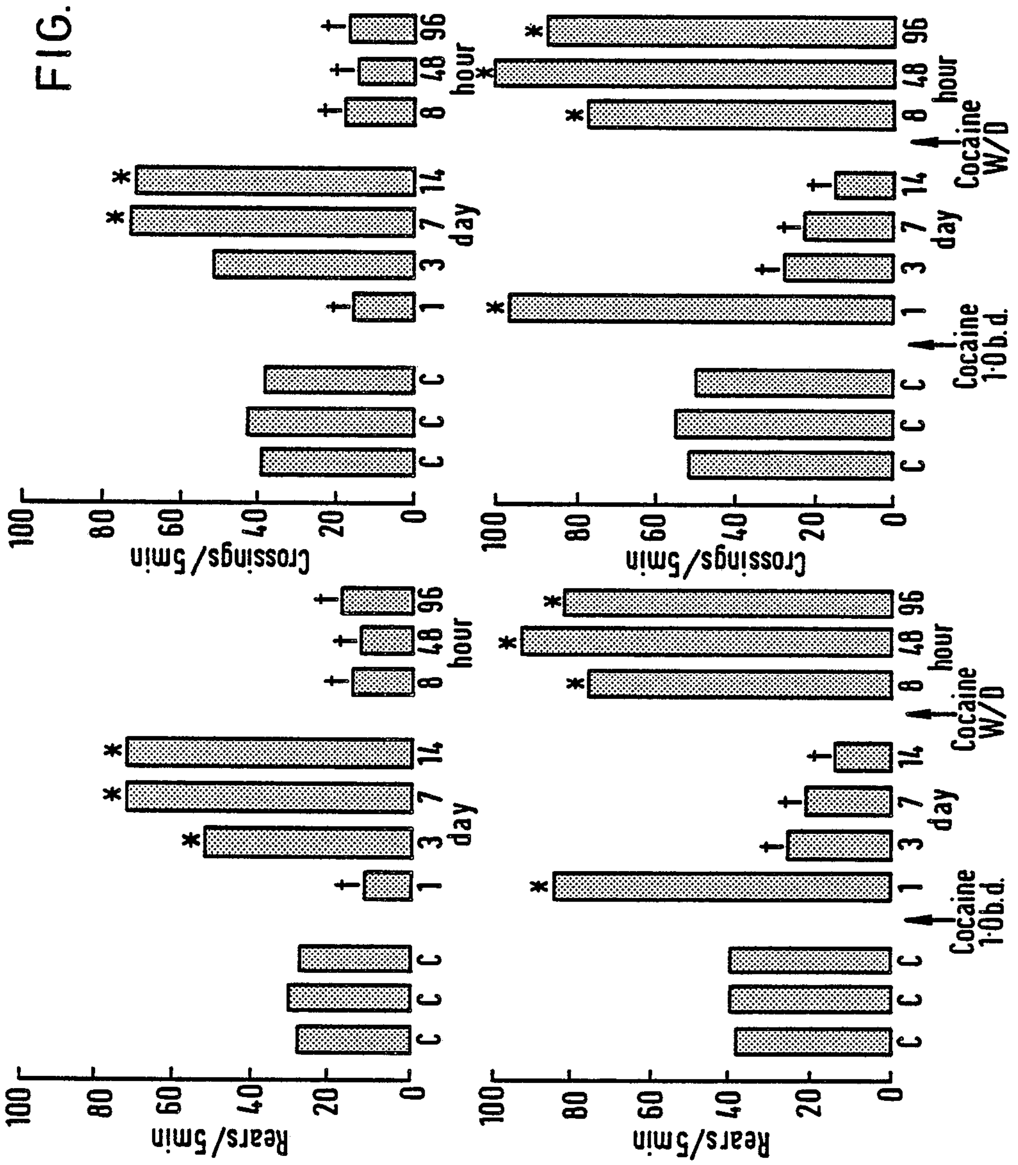
Rears/5min
Crossings/5min
C
1
3
7
14
day
8
48
96
hour
Cocaine 1·0b.d.
Cocaine W/D
100
80
60
40
20
0

Neu eingereicht / Newly filed
Nouvellement déposé
(R 35)



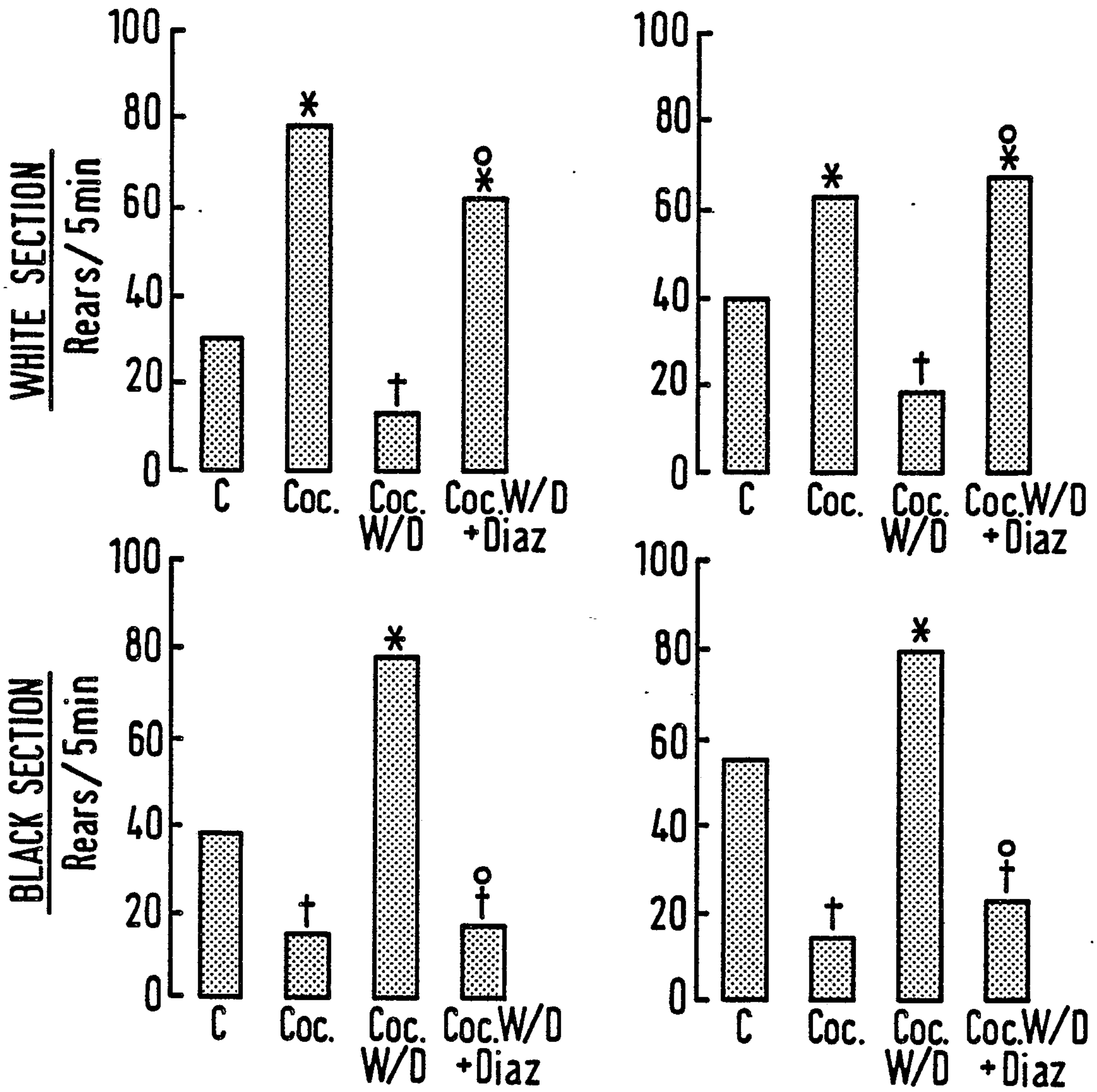


FIG. 8.

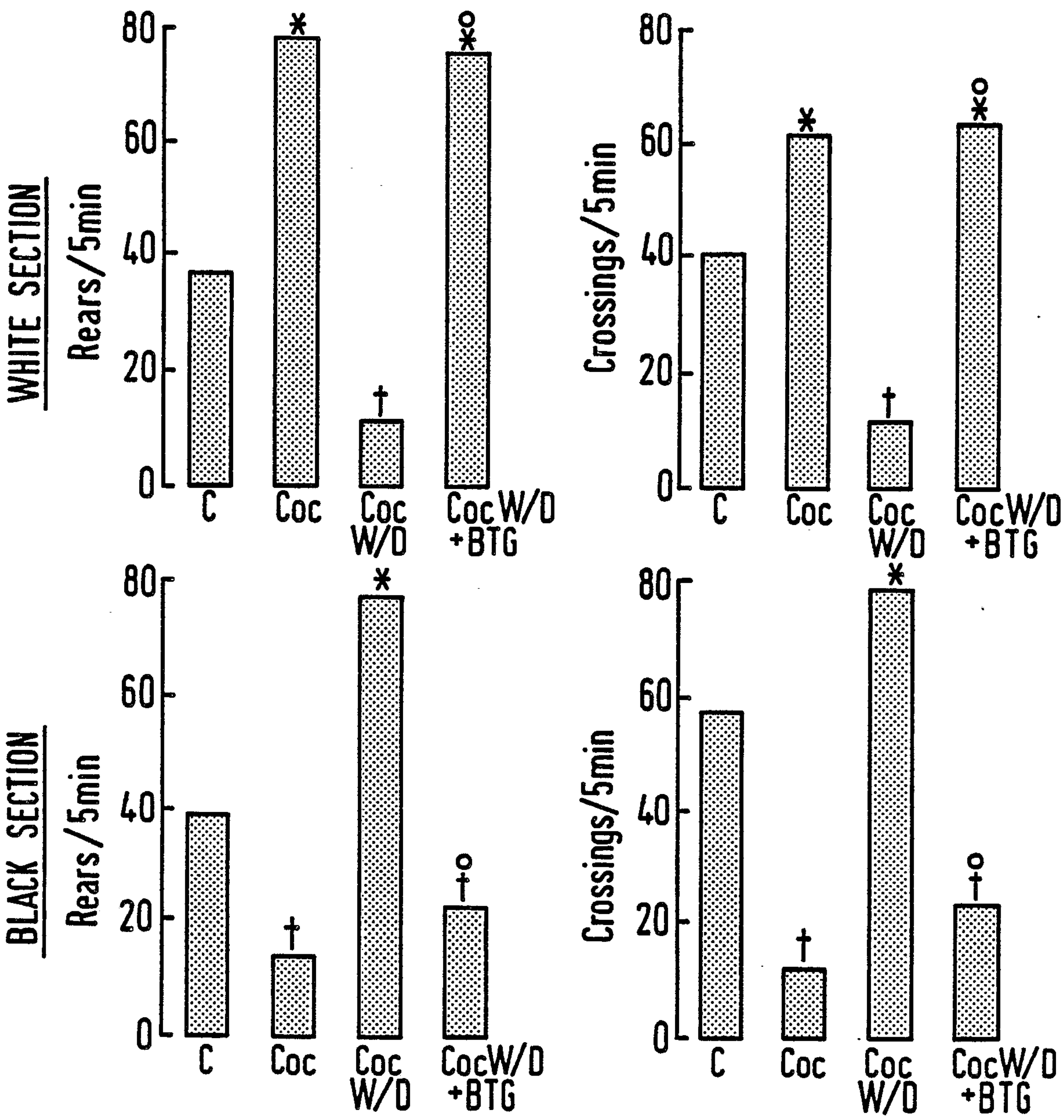
WHITE SECTION
Rears/5min
Crossings/5min
BLACK SECTION
Rears/5min
Crossings/5min
0
20
40
60
80
C
Coc
Coc W/D
CocW/D +BTG

FIG. 9.

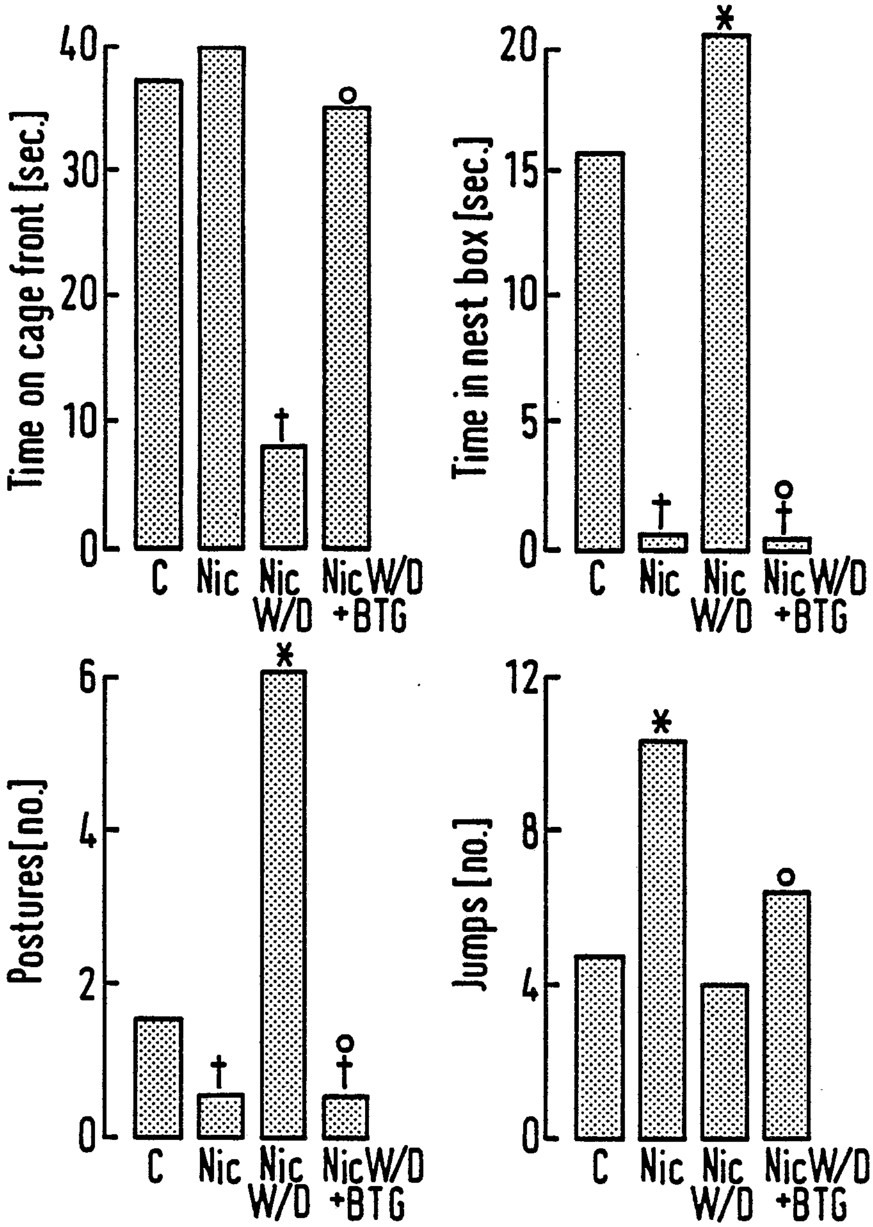
Time on cage front [sec.]
40
30
20
10
0
C
Nic
Nic W/D
Nic W/D +BTG
Time in nest box [sec.]
20
15
10
5
0
C
Nic
Nic W/D
Nic W/D +BTG
Postures [no.]
6
4
2
0
C
Nic
Nic W/D
Nic W/D +BTG
Jumps [no.]
12
8
4
0
C
Nic
Nic W/D
Nic W/D +BTG

FIG. 10.